# EUROPEAN PATENT SPECIFICATION

(11) **EP 3 281 622 B1**
(45) Date of publication and mention of the grant of the patent: **02.06.2021**
(21) Application number: 17184619.9
(22) Date of filing: 03.08.2017
(51) Int. Cl.: A61K 8/81, A61Q 19/10, A61K 8/04

(54) **CLEANING CREAM CONTAINING PVA PARTICLES AND PRODUCING METHOD THEREOF**
REINIGUNGSCREME MIT PVA-TEILCHEN UND HERSTELLUNGSVERFAHREN DAFÜR
CRÈME DE NETTOYAGE CONTENANT DES PARTICULES DE PVA ET SON PROCÉDÉ DE PRODUCTION

(30) Priority: 11.08.2016 TW 105125532
(43) Date of publication of application: 14.02.2018
(73) Proprietor: Jyun He Technology Limited Company, Miaoli County 350 (TW)
(72) Inventor: YAN, HONG-CHANG, Changhua County 504 (TW); LU, YOU-HONG, Miaoli County 350 (TW); LIN, FANG-YUN, Miaoli County 350 (TW)
(74) Representative: Cabinet Chaillot

(56) References cited:
- DE-A1- 19 751 954
- GB-A- 654 211
- GB-A- 680 207
- JP-A- 2003 012 436
- US-A- 3 442 845

## Description

### 1. Field of the Invention

The present invention relates to a cleaning cream, and more particularly to a cleaning cream containing water soluble polymer PVA particles.

### 2. Description of Related Art

Cleaning products that use for exfoliating or whitening skin and teeth are normally contained tiny plastic particles made of Polyethylene (PE) or Polypropylene (PP). When applying the cleaning products, the PE or PP made plastic particles are able to remove dead skin or teeth tartar by rubbing user's skin or teeth.

Because of above mentioned advantages of the PE or PP made plastic particles, more and more applications are introduced to the market. As time goes by, the PE or PP made plastic particles are secretly damaging our health and our treasure planet.

In order to achieve better function and texture, the PE or PP made plastic particles are usually produced with tiny size and added into cleaning products in great deal amount. According to the statistics, at least three hundred and sixty thousands of tiny PE or PP made plastic particles are added into a bottle of facial cleaning cream. It means that tens of thousands of the tiny PE or PP made plastic particles are drained into sewers with contaminated water. Since the PE or PP made plastic particles are too tiny to be collected by water purification plant, this PE or PP made plastic particles will enter into the ocean and our environment eventually. The non-degradable PE or PP made plastic particles then become food for sea creatures or animals and remain in its body for lifespan. After eating this contaminated sea creatures or animals, the PE or PP made plastic particles will transfer to human body and affect human health.

Although some grained natural fiber were used as replacement, there still some limitations like high cost and difficulty of controlling particle size lead grained natural fiber hard to be wildly used as replacement.

To overcome the shortcomings, the present invention provides a cleaning cream containing water soluble polymer PVA particles to mitigate or obviate the aforementioned problems.

In this context, the document GB680207 is relevant.

### SUMMARY OF THE INVENTION

The main objective of the present invention is to provide a cleaning cream containing water soluble polymer PVA particles comprising: water soluble polymer PVA particles being uniformly dispersed in a cleaning cream. The cleaning cream comprises at least a surfactant and the moisture content of the cleaning cream is at the range of 0.1-23 wt%. The water soluble polymer PVA particles comprise water soluble polymer PVA 50-95 wt% and glycerine 0.5-50 wt% in which molecular weight of water soluble polymer PVA is ranging from 110,000 to 120,000 Dalton. The water soluble polymer PVA particles remain in particle form permanently in the cleaning cream.

Water soluble polymer PVA particles of the present invention are able to stay as particle form in the cleaning cream that contained certain quantity of water. The present invention may provide excellent exfoliation and whitening functions when applied to human body. After draining into sewers with contaminated water, the water soluble polymer PVA particles of the present invention will be degraded or dissolved by great amount of water which may solve the problems that conventional PE or PP made plastic particles are not degradable in the environment.

The water soluble polymer PVA particles of the present invention may be manufactured by conventional plastic processing process. The cost will be low and the particle size will be easily controlled by the present invention.

Other objectives, advantages and novel features of the invention will become more apparent from the following detailed description when taken in conjunction with the accompanying drawings.

### IN THE DRAWINGS:

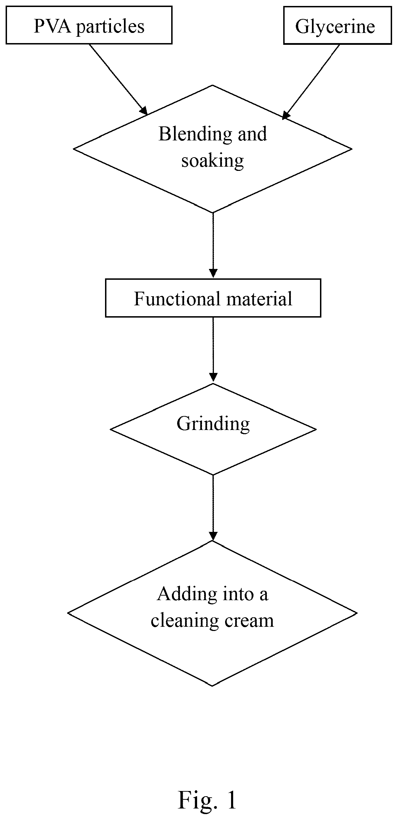

Fig. 1 is a flow chart for producing water soluble polymer PVA particles in accordance with the present invention.

### DETAILED DESCRIPTION OF THE PREFERRED EMBODIMENT

With reference to Fig. 1, a first embodiment for producing cleaning cream containing water soluble polymer PVA particles of the present invention comprises steps of:
blending water soluble polymer PVA particles and glycerine by a high speed mixer letting water soluble polymer PVA particles being completely soaked with the glycerine, wherein the water soluble polymer PVA particles contain water soluble polymer PVA 50-95 wt% and glycerine 0.5-50 wt% after soaking with the glycerine;
adding a functional material 0.5∼4.5 wt% while blending;
grinding the water soluble polymer PVA particles to smooth its surfaces; and
mixing the water soluble polymer PVA particles with a cleaning cream which the moisture content of the cleaning cream is at the range of 0.1∼23 wt% and the cleaning cream contained at least a surfactant. The water soluble polymer PVA particles mixing in the cleaning cream are preferred at range of 0.5∼5wt%.

Particle size of unprocessed water soluble polymer PVA particle is around 100 mesh (150*µ*m) which is incredibly hard and having sharp edges on its surface. After soaking with the glycerine, the particle size of water soluble polymer PVA particles will increase to 70∼80 mesh (180∼220*µ*m) and become much softer and smoother. This may provide comfort and better quality when applied to human body without causing any scratch or even injury.

The functional material as mentioned above may be but not limited to colorant, fragrant or essence. The cleaning cream of the present invention may be a cream, gel or paste. The surfactant in the cleaning cream may be but not limited to anionic surfactant, cationic surfactant, nonionic surfactant or ampholytic surfactant. Beside the surfactant, the cleaning cream may further contain additives as desired like chelating agent or preservative.

PVA is water soluble polymer. It will be dissolved or degraded in an environment contained great amount of water or moisture. In order to keep the water soluble polymer PVA particles as complete particle form in the cleaning cream contained with water/moisture, the molecular weight of water soluble polymer PVA of the present invention is preferred at the range of 110,000∼ 120,000 D (Dalton, D). As the molecular weight increased, the viscosity of water soluble polymer PVA becomes higher and the water soluble property of water soluble polymer PVA otherwise becomes lower. Hence, the viscosity, water-solubility and texture of water soluble polymer PVA in the present invention may be controlled by using water soluble polymer PVA with different molecular weights.

On the other hand, water soluble polymer PVA with molecular weight at the range of 110,000∼120,000 D has better absorption ability to the glycerine which will end up with products with excellent quality and texture. The water soluble polymer PVA particles of the present invention are able to maintain stable in the cleaning cream. When applied to human body, the water soluble polymer PVA particles of the present invention are able to remove dead skin or teeth tartar without causing any scratch or injury. After draining to the sewer, the water soluble polymer PVA particles will be dissolved or degraded by water which won't cause any damage or contamination to the ocean or the environment.

The cleaning products include kinds of preparations with different use methods. To apply directly on the skin or teeth and to apply by forming foam with adding a little bit of water are two most commonly use methods. The foam will contain water/moisture up to 50∼60wt% which is higher than the cleaning cream of the present invention (23 wt%). The water soluble polymer PVA particles of the present invention is still able to keep as complete particle form for at least 2-5 minutes to maintain its function. This may credit to the molecular weight of the water soluble polymer PVA as mentioned above.

Main materials of the present invention only contain water soluble polymer PVA and glycerine. These two materials are considered eco-friendly and safe to human body. It will also cause no harm to sea creatures or animals even being discharge to the ocean or environment.

With reference to below chart 1, samples of the present invention are provided after produced by above mentioned producing method. Chart 1 shows the content of glycerine in water soluble polymer PVA particles, expanded size of water soluble polymer PVA particles and appearance and texture of water soluble polymer PVA particles after soaking with glycerine.

**Chart 1**

| | Glycerine (wt%) | Expanded size (times) | Appearance and texture |
|---|---|---|---|
| Sample 1 | 20 | 4 times bigger than unprocessed water soluble polymer PVA particles | Appearance: round, small |
| | | | Texture: hard |
| | | | Application: Suitable for the cleaning cream like toothpaste or exfoliating product for foot. |
| Sample 2 | 30 | 6 times bigger than unprocessed water soluble polymer PVA particles | Appearance: round, moderate |
| | | | Texture: moderate |
| | | | Application: Suitable for most cleaning creams. |
| Sample 3 | 35 | 7 times bigger than unprocessed water soluble polymer PVA particles | Appearance: round, big |
| | | | Texture: soft |
| | | | Application: Suitable for the cleaning cream like exfoliating or whitening products for face or body. |

According to description above, water soluble polymer PVA particles of the present invention are able to stay as particle form in the cleaning cream that contained certain quantity of water. The present invention may provide excellent exfoliation and whitening functions when applied to human body. After draining into sewers with contaminated water, the water soluble polymer PVA particles of the present invention will be degraded or dissolved by great amount of water which may solve the problems that conventional PE or PP made plastic particles are not degradable in the environment.

The water soluble polymer PVA particles of the present invention may be manufactured by conventional plastic processing process. The cost will be low and the particle size will be easily controlled by the present invention.

## Claims

1. A cleaning cream containing water soluble polymer PVA particles **characterized in that** the cleaning cream comprising:
water soluble polymer PVA particles being uniformly dispersed in a cleaning cream;
the cleaning cream having at least a surfactant and a moisture content of the cleaning cream being at the range of 0.1∼23 wt%;
the water soluble polymer PVA particles being mixed in the cleaning cream at a range of 0.5∼5wt%;
the water soluble polymer PVA particles having water soluble polymer PVA 50∼95 wt% and glycerine 0.5∼50 wt% in which molecular weight of water soluble polymer PVA being ranging from 110,000 to 120,000 Dalton;
the particle size of water soluble polymer PVA particles are in a range of 180-220 µm (70∼80 mesh) after soaking with glycerine; and
the water soluble polymer PVA particles remaining in particle form in the cleaning cream.

2. The cleaning cream containing water soluble polymer PVA particles as claimed in claim 1, wherein the cleaning cream contains chelating agent or preservative.

3. The cleaning cream containing water soluble polymer PVA particles as claimed in claim 1, wherein the surfactant is anionic surfactant, cationic surfactant, nonionic surfactant or ampholytic surfactant.

4. The cleaning cream containing water soluble polymer PVA particles as claimed in claim 1, wherein the water soluble polymer PVA particles contain a functional material 0.5∼4.5 wt%.

5. The cleaning cream containing water soluble polymer PVA particles as claimed in claim 4, wherein the functional material is colorant, fragrant or essence.

## Patentansprüche

1. Reinigungscreme, die wasserlösliche Polymer-PVA-Partikel enthält, **dadurch gekennzeichnet, dass** die Reinigungscreme umfasst:
wasserlösliche Polymer-PVA-Partikel, die in einer Reinigungscreme gleichmäßig dispergiert sind;
wobei die Reinigungscreme zumindest ein Tensid enthält und ein Feuchtigkeitsgehalt in der Reinigungscreme im Bereich von 0,1 ∼ 23 Gew.-% liegt;
wobei die wasserlöslichen Polymer-PVA-Partikel in einem Bereich von 0,5 ∼ 5 Gew.-% in der Reinigungscreme vermischt sind;
wobei die wasserlöslichen Polymer-PVA-Partikel wasserlösliches Polymer-PVA zu 50 ∼ 95 Gew.-% und Glycerin zu 0,5 ∼ 50 Gew.-% aufweisen, wobei das Molekulargewicht von wasserlöslichem Polymer-PVA im Bereich von 110.000 bis 120.000 Dalton liegt;
wobei die Partikelgröße von wasserlöslichen Polymer-PVA-Partikeln nach Tränken mit Glycerin in einem Bereich von 180 ∼ 220 µm (70 ∼ 80 Mesh) liegt; und
wobei die wasserlöslichen Polymer-PVA-Partikel in der Reinigungscreme in Partikelform bleiben.

2. Reinigungscreme, die wasserlösliche Polymer-PVA-Partikel nach Anspruch 1 enthält, wobei die Reinigungscreme Chelatbildner oder Konservierungsmittel enthält.

3. Reinigungscreme, die wasserlösliche Polymer-PVA-Partikel nach Anspruch 1 enthält, wobei das Tensid ein anionisches Tensid, kationisches Tensid, nichtionisches Tensid oder ampholytisches Tensid ist.

4. Reinigungscreme, die wasserlösliche Polymer-PVA-Partikel nach Anspruch 1 enthält, wobei die wasserlöslichen Polymer-PVA-Partikel ein Funktionsmaterial zu 0,5 ∼ 4,5 Gew.-% enthalten.

5. Reinigungscreme, die wasserlösliche Polymer-PVA-Partikel nach Anspruch 4 enthält, wobei das Funktionsmaterial ein Farbstoff, ein Duftstoff oder eine Essenz ist.

## Revendications

1. Crème nettoyante contenant des particules de PVA polymère soluble dans l'eau, **caractérisée par le fait que** la crème nettoyante comprend :
des particules de PVA polymère soluble dans l'eau, qui sont dispersées uniformément dans une crème nettoyante ;
la crème nettoyante ayant au moins un tensio-actif et une teneur en humidité de la crème nettoyante étant dans la plage de 0,1 ∼ 23 % en poids ;
les particules de PVA polymère soluble dans l'eau étant mélangées dans la crème nettoyante dans une plage de 0,5 ∼ 5 % en poids ;
les particules de PVA polymère soluble dans l'eau ayant de 50 ∼ 95 % en poids de PVA polymère soluble dans l'eau et de 0,5 ∼ 50 % en poids de glycérine, la masse moléculaire du PVA polymère soluble dans l'eau se situant dans la plage de 110000 à 120000 daltons ;
la dimension de particule des particules de PVA polymère soluble dans l'eau étant dans une plage de 180 ∼ 220 µm (70 ∼ 80 mesh) après trempage avec de la glycérine ; et
les particules de PVA polymère soluble dans l'eau restant sous forme particulaire dans la crème nettoyante.

2. Crème nettoyante contenant des particules de PVA polymère soluble dans l'eau, selon la revendication 1, dans laquelle la crème nettoyante contient un agent chélatant ou un conservateur.

3. Crème nettoyante contenant des particules de PVA polymère soluble dans l'eau, selon la revendication 1, dans laquelle le tensio-actif est un tensio-actif anionique, un tensio-actif cationique, un tensio-actif non-ionique ou un tensio-actif ampholyte.

4. Crème nettoyante contenant des particules de PVA polymère soluble dans l'eau, selon la revendication 1, dans laquelle les particules de PVA polymère soluble dans l'eau contiennent de 0,5 ∼ 4,5 % en poids d'une matière fonctionnelle.

5. Crème nettoyante contenant des particules de PVA polymère soluble dans l'eau, selon la revendication 4, dans laquelle la matière fonctionnelle est un colorant, un parfum ou une essence.
